# EUROPEAN PATENT APPLICATION

(11) **EP 4 286 075 A1**
(43) Date of publication of application: **06.12.2023**
(21) Application number: 23175003.5
(22) Date of filing: 24.05.2023
(51) Int. Cl.: B22D 46/00, G06F 30/20

(54) **PROCESS DESIGN FOR CASTING COMPONENTS**

(71) Applicant: MAGMA Giessereitechnologie GmbH, 52072 Aachen (DE)
(72) Inventor: STURM, Jörg C., 52072 Aachen (DE); WIEDEMANN, Niklas, 52146 Würselen (DE); OLOFSSON, Jakob, 55313 Jönköping (SE)
(74) Representative: Nordic Patent Service A/S

(57) **Abstract**

A method for predicting local mechanical properties of a casting by executing a casting process simulation (2) to predict local microstructures (6) and local defects (7) at each part of the casting as a function of a set of input variables (1); calculating local microstructure based mechanical properties (8) as a function of the local microstructures (6), and a local damage factor (9) as a function of the local defects (7); and applying the local damage factor (9) to the local microstructure based mechanical properties (8) to determine adjusted local mechanical properties (10) for each part of the object. The method includes statistical analysis for a single cast part or a virtual Design of Experiments (19) using parametrised sets of input variables (18) to determine local defect probability (23) and process capability (24) for each part of the casting.

## Description

### TECHNICAL FIELD

The disclosure relates to processes that involve casting an object in a cavity in a mould. In particular, the disclosure relates to improving such casting processes by predicting local properties of the casting through integrated casting process simulation for optimizing cast object design during design phase and applying statistical analysis for optimizing mould and tool design and casting process conditions during production.

### BACKGROUND

Casting is a manufacturing process by which a liquid material is poured into a mould, which contains a hollow cavity of the desired shape. Dependent on local heat exchange with its environment (e.g. mould, air) the liquid material is cooling down and solidifying in accordance with material specific phase transformation physics. The solidified part is also known as a casting, which is ejected or broken out of the mould to complete the process. Casting is most often used for producing complex shapes that would otherwise be difficult or uneconomical to be produced by other methods. If the casting material is a metal, this metal material is heated-up to a pouring temperature that is higher than the melting temperature and poured into the mould, which may also include runners and risers that enable a controlled filling and solidification of the casting. The metal is then cooling in the mould, the metal solidifies, and the solidified part (the casting) is removed from the mould at a process and material specific metal temperature or process time. Subsequent operations remove excess material required by the casting process (such as the runners and risers).

A simulation of the metal casting process may use multiple techniques including numerical methods to calculate cast component quality considering mould filling, solidification and further cooling, and may provide a quantitative prediction of casting mechanical properties at each point in time during an entire casting process and additional processing steps such as heat treatment. Thus, such simulations can describe the quality of a cast component up-front before production starts. The casting methoding can then be designed with respect to the required component quality and properties. This has benefits beyond a reduction in pre-production sampling, as the precise layout of the complete casting system also leads to energy, material, and tooling savings. The simulation may support product design during design and/or manufacturing stage in determining the casting method, including mould and tool making, as well as subsequent heat treatment and finishing. This may save costs, resources and time along the entire casting manufacturing route.

In particular, for the design of castings for a given part a specific service life needs to be considered. For this, typically the mechanical properties of the material based on given standards are used. Casting properties are related to the basic material and the process conditions for producing the part. In general, there are two different main parameters that influence the final mechanical properties: 1. The local microstructure in the cast part that is determined by the alloy composition, the metal treatment and the local solidification conditions; 2. the presence or absence of local defects that depend on the process layout and the process conditions. Both the local microstructure and the local defects determine the final properties of the part.

During casting design, certain quality requirements are specified to be met during manufacture. These requirements are expressed in terms of property levels for specific areas of the casting that are meeting the expected service loads. This is done to minimise the risk of failure in service. Since the final process conditions are not known during design, the relationship between process, microstructure and final properties is considered a risk. This uncertainty results in a safety factor that is applied to the part design and reduces the optimum potential of the cast material. The result is a part that is usually too heavy for the required service loads.

To realize more optimized lightweight casting designs, taking advantage of the consideration of locally varying properties resulting from a process simulation and use in stress analysis is proposed. This requires that the expected minimum local properties in the casting can be statistically proven at the design stage without experimental validation.

In addition, when the casting part design is fixed, decisions regarding the applied methoding and mould or tool design and decisions on settings for robust process conditions are required. This increases the variety of parameters influencing part quality and related mechanical properties significantly. To realize robust mould and tool designs and process conditions, statistically proven information is needed regarding the influencing parameters on the expected final part properties. The objective is to determine optimal nominal operating points and robust process windows for achieving reliably the required mechanical properties of the part relative to the specification. This information allows a moulding and process design based on expected part quality. It also allows manufacturing to establish operating conditions that will help ensure a seamless and short ramp-up phase. It also allows the manufacturer to provide quantitative information on the expected reliability of the parts before the first casting is produced.

Some commercially available simulation software solutions can provide means for predicting local mechanical properties of an object to be cast to a limited extent, such as providing information about possible porosity formation during the metal casting process or about mechanical properties based on the expected microstructure. However, these known simulation software solutions have not yet been able to provide robust statistical evidence of the varying local properties of a given part area of an object to be cast in a way that the obtained information can be used in order to design robust cast parts as well as to optimize the overall manufacturing process.

### SUMMARY

It is an object to provide an improved method and system for improving a process of casting an object by predicting local properties of the casting that overcomes or at least reduces the problems mentioned above.

The foregoing and other objects are achieved by the features of the independent claims. Further implementation forms are apparent from the dependent claims, the description and the figures.

According to a first aspect, there is provided a computer-implemented method for improving a process of casting an object by predicting local properties of the casting, the method comprising obtaining input variables defining at least one of the designs of the casting, at least one of the designs of the moulding or tooling to be used for casting, the composition of the casting material, any pre-treatment of the casting material, and casting and post treatment process conditions; executing a simulation of the casting process on a computing device using a numerical model of the casting process, the numerical model being configured to predict local microstructures of each part of the casting and local defects mapped to the design of the casting, as a function of the input variables; calculating local microstructure based mechanical properties of each part of the casting as a function of the local microstructures; calculating a local damage factor for each part of the casting as a function of the local defects; and calculating adjusted local mechanical properties of each part of the casting as a combined function of the local microstructure based mechanical properties and the local damage factor.

In a possible implementation form of the first aspect, executing the simulation of the casting process comprises using an integrated microstructure prediction model of mould filling and solidification during casting.

In an embodiment, the integrated microstructure prediction model comprises a numerical model integrating equations defining phase formation, solidification kinetics, segregation, latent heat and solidification, and thermal equilibrium of the mould filling and solidification processes.

In an embodiment, the integrated microstructure prediction model provides output about local microstructures that comprise at least one of local information about grain size, phase distributions for primary and intermetallic phases, and related phase sizes of a given part of the casting.

In an embodiment, the integrated microstructure prediction model calculates the local microstructure based mechanical properties and related local stress-strain curves describing the mechanical performance, such as yield strength, tensile strength and elongation of a given part of the casting.

In a further possible implementation form of the first aspect, calculating the adjusted local mechanical properties comprises applying respective local damage factors to local stress-strain curves at each part of the casting to calculate a local reduction in the respective mechanical performance at each part.

In an embodiment, calculating adjusted local mechanical properties comprises determining a simulated local defect map by mapping the simulated local defects to the geometrical model of the casting, defining one or more evaluation areas in the geometrical model that may reflect virtual local tensile strength tests on the local defect map, and applying respective local defects to the local tensile strength tests to determine local stress-strain curves.

In a further possible implementation form of the first aspect the predicted local defects comprise attributes of a given part of the casting that can reduce the material performance locally, such as entrapped air and gases, cold shuts, weld lines, porosity, and other detrimental inclusions.

In an embodiment the local damage factor for each part of the casting is calculated as a function of the size and other characteristics of the local defects.

In a further possible implementation form of the first aspect the method further comprises obtaining a set of fixed input variables defining a nominal operating point by nominal process conditions; obtaining a process robustness parameter defining the reliability of the nominal process conditions; selecting at least one evaluation area of the casting; and determining a design capability for the at least one evaluation area by executing a simulation of the casting process using the numerical model of the casting process for each of the fixed input variables to predict a distribution of local microstructures and a distribution of local defects at every point of the evaluation area; calculating a distribution of local microstructure based mechanical properties at every point of the evaluation area as a function of the distribution of local microstructures; calculating a distribution of local damage factors at every point of the evaluation area as a function of the distribution of local defects; and calculating a distribution of adjusted local mechanical properties at every point of the evaluation area as a combined function of the distribution of local microstructure based mechanical properties and the distribution of local damage factors.

In an embodiment the evaluation area corresponds to the entire geometrical model of the casting.

In another embodiment each evaluation area corresponds to sub-regions of the casting based on different respective load levels to be applied.

In an embodiment the different load levels correspond to static, dynamic, and/or crash-related load cases.

In an embodiment the method further comprises obtaining a set of fixed input variables defining nominal process conditions based on a nominal operating point.

In one embodiment the set of fixed input variables define composition of the casting material, current part design, tool design to be used for casting, and nominal process conditions for filling and cooling.

In an embodiment determining the design capability comprises determining the adjusted local tensile strength in each evaluation area and applying a statistical assessment on each evaluation area based on local stress-strain curves corresponding to the respective local tensile strength samples defining variations in adjusted local mechanical properties based on variations in one of the set of input variables.

In a further possible implementation form of the first aspect determining the design capability for the at least one evaluation area comprises determining a minimum of expected local mechanical properties for the at least one evaluation area of the casting based on the distribution of adjusted local mechanical properties at every point of the evaluation area.

In an embodiment determining the design capability further comprises calculating a distribution and probability of expected local mechanical properties for the at least one evaluation area of the casting based on the distribution of adjusted local mechanical properties at every point of the evaluation area.

In a further possible implementation form of the first aspect the method further comprises mapping at least one of the determined minimum of expected local mechanical properties, or the distribution and probability of expected local mechanical properties onto a Finite Element model defining the geometry of the casting and the at least one evaluation area; analysing the Finite Element model using FEA analysis for evaluating mechanical performance based on user requirements defining local loads to be applied to the at least one evaluation area.

In an embodiment the method further comprises adjusting the Finite Element model and/or determining a set of adjusted input variables to fulfil the user requirements.

In a further possible implementation form of the first aspect the method further comprises obtaining a parametrised set of input variables defining variations in a plurality of the input variables; executing a Design of Experiments by executing multiple simulations of the casting process using the numerical model of the casting process for possible combinations of the parametrised set of input variables to predict variations in local microstructures and variations in local defects at each part of the casting; and determining a statistical strength assessment for the parametrised set of input variables in each part of the casting by calculating variations in local microstructure based mechanical properties at each part of the casting as a function of the variations in local microstructures; calculating variations in local damage factors at each part of the casting as a function of the variations in local defects; and calculating variations in adjusted local mechanical properties as a combined function of the variations in local microstructure based mechanical properties and the variations in local damage factors.

In a further possible implementation form of the first aspect the method comprises determining a local defect probability for each of the parametrised set of input variables in each part of the casting as a function the variations in local defects.

In a further possible implementation form of the first aspect the method comprises a full factorial assessment by executing simulations of the casting process for all possible combinations of the parametrised set of input variables.

In another possible implementation form of the first aspect the method comprises a limited assessment by applying a statistically supported reduction of the number of the parametrised set of input variables and executing a number of simulations of the casting process for a reduced parametrised set of input variables and a set of fixed input variables.

In a further possible implementation form of the first aspect the method further comprises determining information about process capability for the parametrised set of input variables for defining a statistical robustness of the casting process by determining a main effect and correlation diagram describing the impact of varied input parameters on the cast part quality, an expected minimum of mechanical properties and a distribution of mechanical properties of the casting based on predefined quality criteria, and/or a robust process window for the parametrised set of input variables.

In an embodiment determining the process capability for the parametrised set of input variables comprises calculating a normal distribution of local mechanical properties for each part of the casting.

In an embodiment determining the robust process window comprises adjusting a process window defined by the parametrised set of input variables by reducing process variations for process robustness.

In an embodiment determining the robust process window comprises adjusting an operating point defined by the parametrised set of input variables to determine an optimal operating point.

In a further possible implementation form of the first aspect the method further comprises assessing the impact of variations in the parametrised set of input variables on the expected part quality and related mechanical properties using the main effect and correlation diagram.

In a further possible implementation form of the first aspect the method further comprises comparing at the minimum of expected mechanical properties and/or the distribution of expected mechanical properties to predefined user requirements regarding quality criteria of the casting; and determining an adjusted parametrised set of input variables to meet the user requirements.

In a further possible implementation form of the first aspect the method further comprises obtaining at least one of the set of fixed input variables or parametrised set of input variables via an input-output interface of the computing device; and outputting the determined result of at least onestep of the performed method to a display of the computing device.

According to a second aspect, there is provided a computer-based system comprising an input-output interface; a display; a non-transitory machine-readable storage medium including a computer program product; and at least one processor operable to execute the program product, interact with the input-output interface and the display, and perform operations according to the methods of any one of the possible implementation forms of the first aspect.

According to a third aspect, there is provided a computer program product, encoded on a non-transitory machine-readable storage medium, operable to cause a processor to perform operations according to the methods of any one of the possible implementation forms of the first aspect.

These and other aspects will be apparent from the embodiment(s) described below.

### BRIEF DESCRIPTION OF THE DRAWINGS

In the following detailed portion of the present disclosure, the aspects, embodiments and implementations will be explained in more detail with reference to the example embodiments shown in the drawings, in which:
Fig. 1 is a flow diagram illustrating a method for predicting local mechanical properties of an object to be cast according to a first aspect.
Fig. 2 is a flow diagram illustrating aspects of the integrated microstructure predicting model for mould filling and solidification during the casting process according to a possible implementation of the first aspect.
Fig. 3 is a flow diagram illustrating the a method of using evaluation areas as virtual local tensile strength tests and determining local stress-strain curves at each part of the object for calculating adjusted local mechanical properties according to a possible implementation of the first aspect.
Fig. 4 is a flow diagram illustrating a method for determining a design capability for evaluation areas of the object to be cast with different load requirements and the feedback of the design capability results as an input for load analysis (FEA) according to a possible implementation of the first aspect.
Fig. 5 is a flow diagram illustrating a method for determining a process capability with sets of parametrised input variables in a virtual Design of Experiments (DoE) approach according to a possible implementation of the first aspect.
Fig. 6 is a scatter diagram illustrating the outcome of a virtual DoE in terms of predicted mechanical properties for the variation of two process parameters.
Fig. 7 is a block diagram illustrating a computer-based system for predicting local mechanical properties of an object to be cast according to a second aspect.

### DETAILED DESCRIPTION

When designing a new cast part, the focus is on the required functions and the design evaluation is based on the load cases to be expected in service life. The cast part has to fulfil multiple functions. Therefore, diverse load cases will be investigated by Finite Element Analysis (FEA), which is a computerized method for predicting how a product reacts to real-world forces, vibration, heat, fluid flow, and other physical effects. FEA can show whether a product will break, wear out, or work the way it was designed, and requires either static, dynamic (durability) and/or crash related input for properties. With respect to the material specification, the design process usually relies on available standards for the given material and process. Deviations from accepted standards of the manufactured part are only accepted under quality system considerations, i.e. certain areas of different loads in the part and related acceptable defects are specified that must be met by manufacturing accordingly. This information is part of the specification sheet during procurement. Depending on the company standards safety factors are added that reduce the allowable load of a cast part in addition for safety reasons.

As a result, the cast part is usually over dimensioned for two reasons: on one hand, using only standard data instead of local properties; and on the other hand, using additional safety factors to cope with unknown local properties.

The innovation described in this application uses the predictions from virtual process simulation (material performance and local defects) and predicts local mechanical properties. It allows to associate given load levels of the part with the predicted local properties that can be met based on the given process conditions during the design process of the cast part. During the design process, the safety factors can be reduced and the expected local properties can be taken into account to reduce the weight of the part.

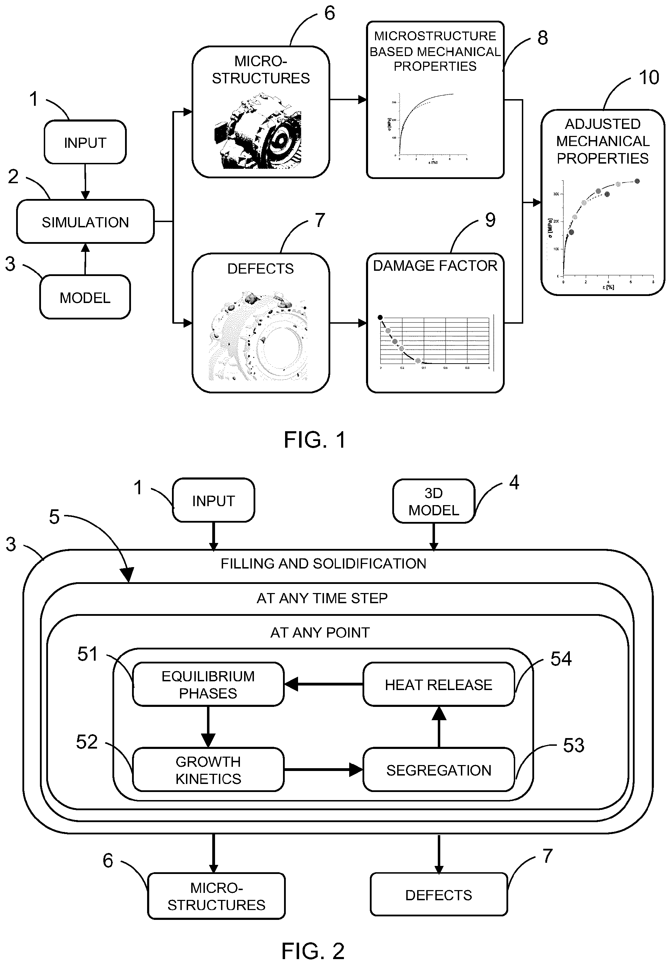

Fig. 1 is a flow diagram illustrating the main steps of the method for improving such a process of designing an object to be cast according to the present disclosure, by predicting adjusted local mechanical properties 10 of the object taking into account both local microstructures 6 as well as local defects 7 in each part of the object.

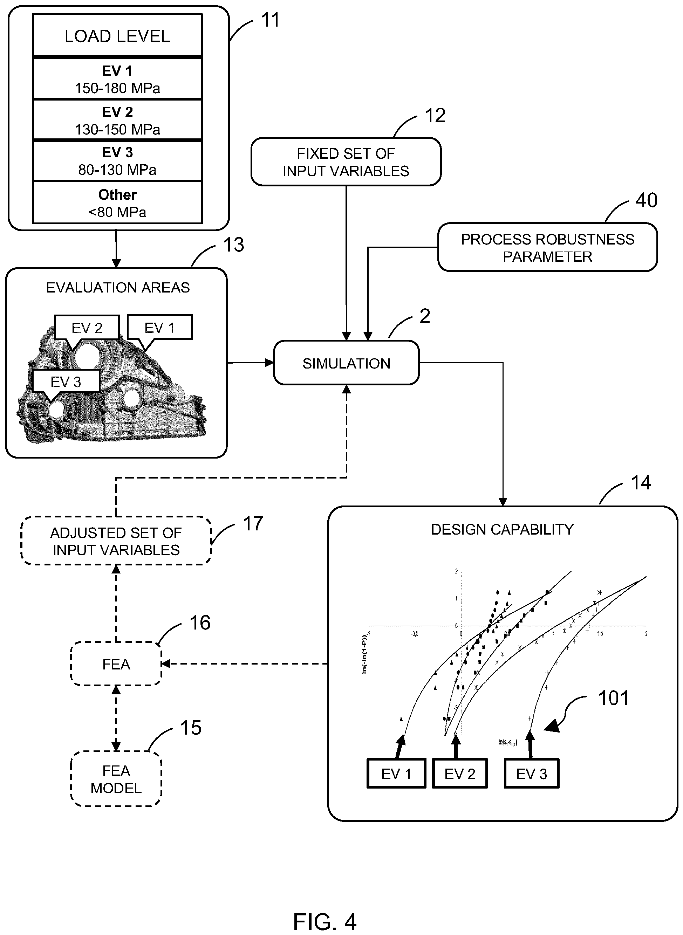

The input for the casting process simulation 2 of casting parts consists of information regarding the part design, the composition of the material chosen, the moulding or tooling design, a set of fixed nominal process conditions for filling and solidification and further cooling and a general process robustness parameter 40, as shown in Fig. 4. These input parameters are defined for the simulation method via input parameters 1.

The above process parameters are nominal and do not consider a scatter or fluctuation inherent in any manufacturing process. The process robustness parameter 40 adds a probability distribution describing the reliability of the process anticipated.

As at this stage final decisions on the mould or tooling design and the process conditions have not been done, assumptions will be made based on previous reference examples or best practice.

In addition, certain evaluation areas 13 or zones can be defined that require a certain load level, as shown in more detail in Fig 4.

The numerical models 3 applied for the simulation 2 are describing the filling and the solidification of the part to be cast. An integrated microstructure prediction model 5 can be applied during filling and solidification, shown more detail in Fig. 2 and explained more below.

The output of these numerical models is twofold: on one hand, predicted local microstructures 6 describing local information about grain size, phase distributions for primary and intermetallic phases, and related phase sizes of a given part of the object to be cast, as well as related material performances in terms of local microstructure based mechanical properties that describe the fundamental behaviour of the material in use.

On the other hand, local defects 7 are also predicted and mapped to the design of the object to be cast, both as a function of the input variables 1. The predicted local defects 7 comprise attributes of a given part of the object to be cast that can reduce the material performance locally, such as entrapped air and gases, cold shuts, weld lines, and porosity.

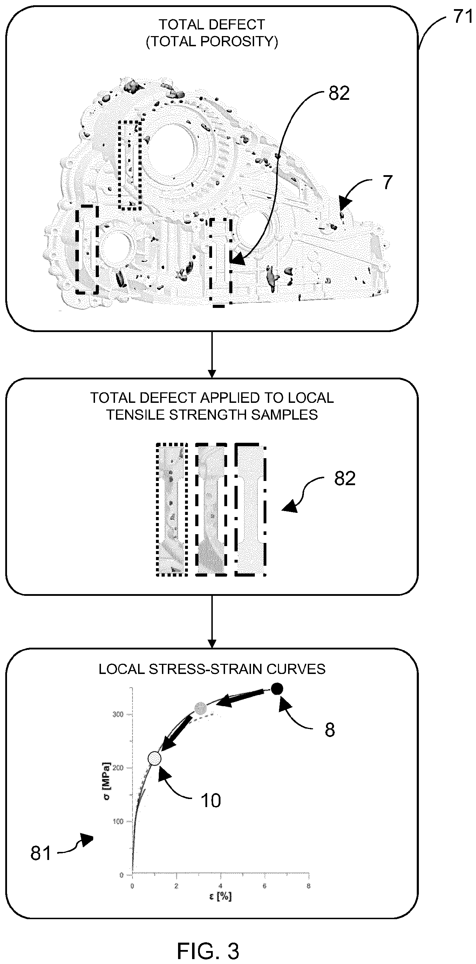

Based on the assessment of the local microstructures 6 the method predicts local microstructure based mechanical properties 8 based on the local microstructures 6 and related local stress-strain curves 81 as shown in more detail in Fig. 3, describing the mechanical performance, such as yield strength, tensile strength and elongation of a given part of the object to be cast.

The predicted local defects 7 are also assessed in terms of their detrimental effect on the material performance by a local damage factor 9. Defects predicted can be such as entrapped air and gases, cold shuts, weld lines porosity and other detrimental inclusions or a combination of the above. This local damage factor 9 can be a function of defect size or other characteristics of the individual defects 7.

The local damage factor 9 is then be applied locally to the predicted microstructure stress-strain curves 81 reducing the optimal material performance to lower values of tensile strength and ductility and thereby arrive at a local weakening in the respective mechanical performance at any location of the part.

The final output of the illustrated flow is an integral assessment of the part as a function of a general process robustness parameter 40 from input 1, the local microstructure 6 and the local defect 7 situation in the form of adjusted local mechanical properties 10 of each part of the object to be cast.

The above methodology can be applied to a single simulation 2 for a given part and process condition, providing statistically secured adjusted local mechanical properties 10 to be used in part design to avoid unnecessary safety margins that lead to increased part weight.

As illustrated in Fig. 2, an integrated microstructure prediction model 5 can be applied during simulation of the filling and solidification 3. The integrated microstructure prediction model 5 provides the link between the input parameters 1 such as material specification and the process conditions, the geometrical 3D-model 4, metal treatment and the resulting local microstructures 6, the local defects 7 and the final microstructure related microstructure based mechanical properties. The integrated microstructure prediction model 5 comprises a numerical model integrating equations defining equilibrium phases being created based on nominal material composition 51, the growth kinetics for all phases 52, the segregation of all elements involved 53, the precipitation of latent heat and solidification 54, for the mould filling and solidification processes at any point in the object to be cast and at any time of the process.

The output of this numerical model are local microstructures (such as phase amounts and size of primary and intermetallic phases), and provides information about local microstructures 6 and local defects 7 occurring in the cast part.

As Fig. 3 illustrates, calculating the adjusted local mechanical properties 10 may comprise determining a local defect map 71 by mapping local defects 7 to the design of the object to be cast, then applying a method to determine the adjusted local tensile strength 81 using tensile test 82 or evaluation areas 13 in the object to be cast based on the local microstructure map 6 and the defect map 71, and applying respective local defects 7 to the local tensile strength test or evaluation areas 82 to determine local stress-strain curves 81.

In particular, the local microstructure based mechanical properties 8 are calculated based on the local microstructures 6 and related local stress-strain curves 81 describing the associated material performance, to which the local damage factors 9 are applied at each part of the object to be cast to calculate a local weakening in the respective mechanical performance at each part of the object.

The method uses the predicted information to calculate adjusted local mechanical properties 10 in the entire cast part for the given conditions, defined in the form of adjusted values of stress-strain curves. These are illustrated in Fig. 3 through adjusted positions of dots along a particular stress-strain curve 81 at an example location of the object to be cast, showing an optimal material performance of the microstructure based mechanical properties 8 (dot in the upper right end of the curve) reduced to lower values of tensile strength and elongation to the adjusted mechanical properties 10 (dots on the left from the optimal performance) at any location of the part.

The above methodology can be applied to a single simulation for a given object and process condition, providing statistically secured local mechanical properties to be used in part design to avoid unnecessary safety margins that lead to increased part weight.

The user can also decide in which sections of the part, so-called evaluation areas (EV) 13 the analysis will be performed, wherein the analysis of all locations in the respective evaluation areas 13 will result in statistically proven minimum mechanical properties that can be used as input for FEA analysis for part performance.

Fig. 4 illustrates a flow diagram for applying the above methodology for such a purpose, namely for determining a design capability 14 for certain evaluation areas 13 of the object to be cast according to a possible implementation of the first disclosure.

In this case the required functions and the layout of the object to be cast are evaluated based on the expected load cases. The object to be cast has to fulfil multiple functions and therefore, diverse load cases will be investigated by FEA that require either static, dynamic (durability) and/or crash related input for properties.

The method thus comprises selecting at least one evaluation area 13 (in this case EV 1, EV 2, EV 3) of the object to be cast according to different user requirements 11, in this case different local loads 11 expected in different parts of the object based on a so-called zone model that assigns specific zones to a given stress level to be considered. However, it is also possible that a single evaluation area 13 corresponds to the entire geometrical 3D-model of the object to be cast.

As described before, at this stage preliminary input for a casting process simulation 2 of the cast part may be defined, consisting of information about the current part design, the composition of the selected material, a first mould or tool design and nominal process conditions for filling and cooling defined as set of fixed input variables 12. As at this stage final decisions on the moulding or tooling design and the process conditions have not been done, assumptions will be made based on previous reference examples or best practice. A process robustness parameter 40 is further defined in addition to the set of fixed input variables 12, adding a probability distribution describing the reliability of the process anticipated.

Subsequently the method automatically evaluates the predefined evaluation areas 13 in the part using the adjusted local mechanical properties 10 and provides statistically based information about the minimum properties and the distribution of properties that can be expected.

In an example, this is done by executing a simulation 2 of the casting process using the numerical model 3 of the casting process to predict a distribution of local microstructures 6 and a distribution of local defects 7 of each part of the evaluation areas (EV1, EV2, EV3) 13; calculating a distribution of local microstructure based mechanical properties 8 at each part of the evaluation areas 13 as a function of the distribution of local microstructures 6; calculating a distribution of local damage factors 9 at each part of the evaluation area 13 as a function of the distribution of local defects 7; and then calculating a distribution of adjusted local mechanical properties 10 at each part of the evaluation area 13 as a combined function of the distribution of local microstructure based mechanical properties 8 and the distribution of local damage factors 9.

The method comprises the evaluation of the above information in the different evaluation areas or throughout the entire object to be cast by statistical means. Output of this statistical evaluation is a design capability 14 comprising a set of minimal local mechanical properties 101 for any evaluation area 13 and its scatter, i.e. a distribution of these local mechanical properties (e.g. yield strength, tensile strength and elongation). This output allows to associate the given load levels of the part with the predicted local properties that can be met based on the given process conditions during the design process.

The information about minimal local mechanical properties 101 can be exported from the process simulation tool to the FEA load analysis 16 as an additional input or optimization of the part design through existing interfaces.

The result is a part design (e.g. a 3D-model 4) that meets the user requirements (i.e. local loads 11) with minimal material consumption. The part design thus benefits from the expected minimum local properties in the defined areas as a function of the design and process and gain additional information about the robustness of his design.

In other words, using this method given uncertainties regarding the tooling layout or process parameter settings can be accepted by the designer as the output compensates these uncertainties by offering additional valuable information about local properties at an early stage, so that the designer can reduce the safety factors and use the expected local properties to reduce the weight of the part.

At a later stage in the product and process development process the manufacturing has to set up the casting process in detail. The primary goal of the manufacturing is to use a casting and gating layout and process conditions that provide robust tooling designs, operating conditions, and a robust process window that can handle the inevitable process variation at an acceptable cost. Since no casting is defect free, the control of defects and their impact on the specified quality levels (in the final mechanical properties) is paramount.

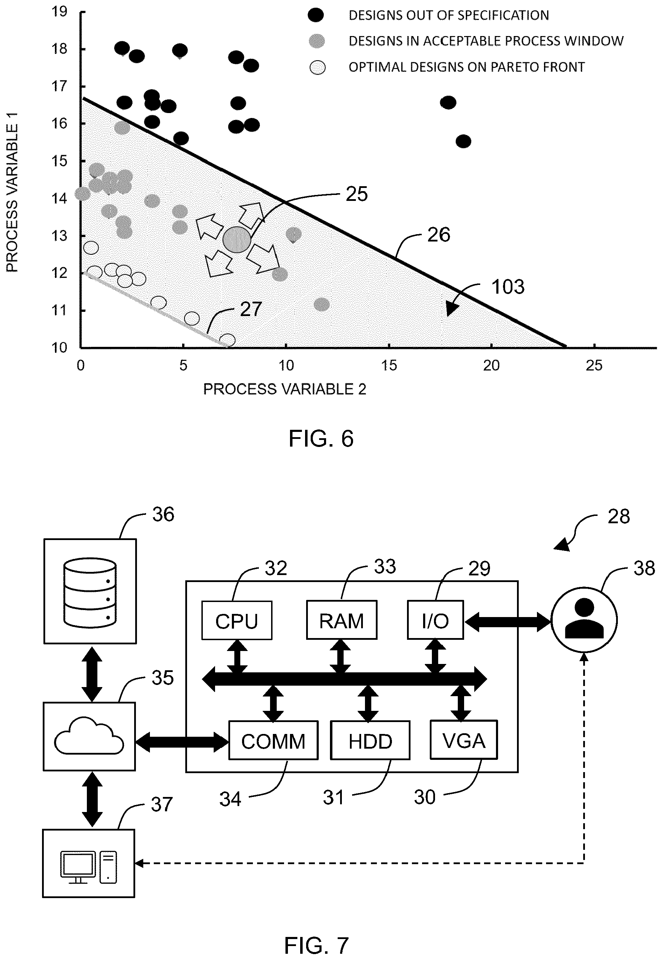

To help in this, the methodology of the disclosure can also be applied in the manufacturing (or generally spoken whoever is responsible for the casting manufacturing process) on a chosen process window or typical process scatter (see also Fig. 6), whereby design and process responsible can be provided with information from a statistical analysis based on a virtual set of experiments using casting process simulation 2 linked to a so-called Design of Experiments 19 (DoE) for a number of designs 1 to N. This allows to assess the impact of the variations on the expected part quality and related mechanical properties, or even to optimize and arrive at optimal operating points and robust process windows for the expected mechanical properties of the part relative to the specification using process simulation as a virtual test bench to meet the specifications from the customer.

The method allows not only to simulate a nominal operating point 25 but also offers the assessment of the influence of the geometry, the material or process variables on the adjusted mechanical properties via parametrization of geometries or definition of variations of selected input variables 1.

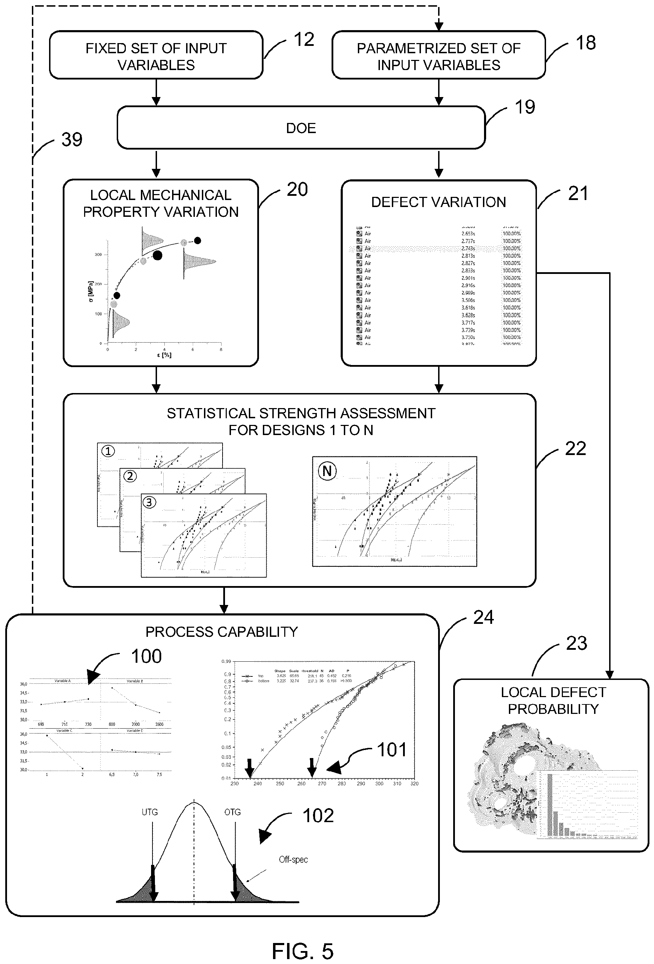

Fig. 5 is a flow diagram illustrating such a method for determining information about the process capability 24 and a local defect probability 23 for parametrised input variables 18 according to a possible implementation of the disclosure. The methodology applied is principally the same as described before with respect to Figs. 1-6, but instead of defining and running a process simulation 2 for a single (e.g. nominal) operating point, the manufacturing may vary any of the part and mould or tool design (example: different gating concepts or cooling layouts), the process window with multiple input variables (example: varied filling conditions such as pouring time and temperature, filling time, mould temperatures) and/or can consider process scatter (e.g. impact on operational breaks on part quality) to define the parametrised set of input variables 18 for executing a virtual Design of Experiments 19, in addition to fixed input variables 12. Depending on the number of parametrised input variables 18, multiple process simulations 2 can be run automatically without human interaction using the numerical model 3 of the casting process for any possible combination of such parametrised input variables 18 for a number of designs 1 to N.

In some cases, this includes running a full factorial assessment by executing a simulation 2 of the casting process for all possible combinations of parametrised input variables 18.

In other cases, the method comprises the reduction of the number of variants to be simulated by statistical methods for the virtual Design of Experiments (using methods such as Reduced Factorial or Sobol), resulting in a reduction of the number of parametrised input variables 18 and executing a simulation 2 of the casting process for the reduced number of parametrised input variables 18 and a set of fixed input variables 12.

Variations in local microstructure based mechanical properties 8 of each part of the casting can then be calculated as a function of the variations in local microstructures 6, as well a variation of local damage factors 9 based on a list of variations in local defects 21.

The initial output is information about the local mechanical property variation in the form of variations in adjusted local mechanical properties 20 as a combined function of variations in local microstructure based mechanical properties 8 and variations in local damage factors 9, and the defect variation 21 as a function of the process or design variation. The different defect levels and distributions and associated mechanical properties for the varied conditions are automatically analysed for all designs 1 to N by built-in statistical tools.

The intermediate result of this process is a statistical strength assessment 22 for each part of the object to be cast as a function the variations 1 to N in local mechanical property variation 20 and the local defect variation 21. The statistical analysis provides information about the lowest properties and their distribution for any of the investigated designs 1 to N.

The method also provides information about a local defect probability 23 or any other predicted quality criterion for any of the investigated designs 1 to N.

The final output for the manufacturer is information about a process capability 24 that provides quantitative data on the correlation between input variables and the expected casting quality (in form of main effect and correlation diagrams 100), the minimum 101 and distribution 102 of expected mechanical properties for the process variation for the defined design or process variation so that the chosen design and process decisions can be assessed, arriving at a statistically proven robustness 103 (in form of a robust process window) to produce reliable parts under all circumstances before the first real part is produced.

The outcome benefits the manufacturer in terms of a safe production start and ramp-up. The available information can also be passed on to the casting user as proof that reliable operating conditions have been chosen.

The above-described process capability information 24 can further help determining a robust process window 103 (grey area between 26 and 27) for the parametrised set of input variables 18 as shown in Fig. 6.

This illustrated scatter diagram describing the output of a virtual Design of Experiments for the example of two process variables. Each dot/point reflects the predicted mechanical property as a function of a combination of process variables 1 and 2. The nominal operating point 25 can be described as either reflecting the set of fixed variables 12 to determine the design capability 14 or as the nominal centre point of a process window for a parametrised set of input variables 18 with a given scatter to assess the process capability 24. The bold line 26 describes the process limits to meet the required specification. Any design exceeding this line will not meet the specification; any design under this line shows results meeting the required specification. For the number N of investigated results the grey line 27 reflects the Pareto frontier, i.e. the limits for the combination of optimal robust process conditions.

In this illustrated example, the method of the disclosure comprises adjusting a process window defined by a fixed set of input variables 12 and a parametrised set of input variables 18 by reducing process variations for process robustness for determining a robust process window 103 in a first step.

Once this robust process window 103 is established, in a next step the operating point 25 defined by the parametrised input variables 18 can be adjusted to determine an optimal operating point 25.

Fig. 7 is a schematic block diagram illustrating an example of a hardware configuration of a computer-based system 28 for predicting local mechanical properties of an object to be cast in accordance with the present disclosure.

The computer-based system may comprise one or more processors (CPU) 32 configured to execute instructions that cause the computer-based system to perform a method according to any of the possible embodiments described above.

The computer-based system may also comprise computer-readable storage medium 31 configured for storing software-based instructions as part of a program product to be executed by the CPU 32.

The computer-based system may also comprise a memory 33 configured for (temporarily) storing data of applications and processes.

The computer-based system may further comprise an input-output interface 29 for user interaction between the system and a user 38, connected to or comprising an input interface (such as a keyboard and/or mouse) for receiving input from a user 38, and an output device such as an electronic display 30 for conveying information to a user 38.

The computer-based system may further comprise a communications interface 34 for communicating with external devices such as a remote client 37 directly or indirectly via a computer network 35.

The mentioned hardware elements within the computer-based system may be connected via an internal bus configured for handling data communication and processing operations.

The computer-based system may further be connected to a database 36 configured for storing data to be used as input for the above-described simulations (such as material properties for cast materials, experimental data, etc.), wherein the type of connection between the two can be direct or indirect. The computer-based system and the database 36 can be both included in the same physical device, connected via the internal bus, or they can be part of physically different devices, and connected via the communication interface 34 either directly, or indirectly via a computer network 35.

The various aspects and implementations have been described in conjunction with various embodiments herein. However, other variations to the disclosed embodiments can be understood and effected by those skilled in the art in practicing the claimed subject-matter, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

The reference signs used in the claims shall not be construed as limiting the scope.

## Claims

1. A computer-implemented method for improving a process of a casting by predicting local mechanical properties of the casting, the method comprising:
obtaining input variables (1) defining at least one of the design of the casting, the design of the moulding or tooling to be used for casting, the composition of casting material, any pre-treatment of the casting material, and casting and post treatment process conditions;
executing a simulation (2) of the casting process on a computing device (28) using a numerical model (3) of said casting process, the numerical model (3) being configured to predict local microstructures (6) of each part of the casting and local defects (7) mapped to the design of the casting, as a function of said input variables (1);
the method further comprising executing by the computing device (28) the steps of:
calculating local microstructure based mechanical properties (8) of each part of the casting as a function of said local microstructures (6);
calculating a local damage factor (9) for each part of the casting as a function of said local defects (7); and
calculating adjusted local mechanical properties (10) of each part of the casting as a combined function of said local microstructure based mechanical properties (8) and said local damage factor (9).

2. The method according to claim 1, wherein executing the simulation (2) of the casting process comprises using an integrated microstructure prediction model (5) of mould filling and solidification during casting.

3. The method according to any one of claims 1 or 2, wherein calculating said adjusted local mechanical properties (10) comprises applying respective local damage factors (9) to local stress-strain curves (81) at each part of the casting to calculate a local weakening in the respective mechanical performance at each part.

4. The method according to any one of claims 1 to 3, wherein the predicted local defects (7) comprise attributes of a given part of the casting that can reduce the material performance locally, such as entrapped air and gases, cold shuts, weld lines, porosity, and other detrimental inclusions.

5. The method according to any one of claims 1 to 4, the method further comprising:
obtaining a set of fixed input variables (12) defining a nominal operating point by nominal process conditions;
obtaining a process robustness parameter (40) defining the reliability of said nominal process conditions;
selecting at least one evaluation area (13) of the casting; and
determining a design capability (14) for said at least one evaluation area (13) by
executing a simulation (2) of the casting process using said numerical model (3) of said casting process for each of said fixed input variables (12) to predict a distribution of local microstructures (6) and a distribution of local defects (7) at every point of the evaluation area (13);
calculating a distribution of local microstructure based mechanical properties (8) at every point of the evaluation area (13) as a function of said distribution of local microstructures (6);
calculating a distribution of local damage factors (9) at every point of the evaluation area (13) as a function of said distribution of local defects (7); and
calculating a distribution of adjusted local mechanical properties (10) at every point of the evaluation area (13) as a combined function of said distribution of local microstructure based mechanical properties (8) and said distribution of local damage factors (9).

6. The method according to claim 5, wherein determining said design capability (14) for said at least one evaluation area (13) comprises determining a minimum of expected local mechanical properties (101) for said at least one evaluation area (13) of the casting based on said distribution of adjusted local mechanical properties (10) at every point of the evaluation area (13).

7. The method according to claim 5 or 6, wherein determining the design capability (14) further comprises calculating a distribution and probability of expected local mechanical properties for said at least one evaluation area (13) of the casting based on said distribution of adjusted local mechanical properties (10) at every point of the evaluation area (13).

8. The method according to claim 6 or 7, the method further comprising:
mapping at least one of said determined minimum of expected local mechanical properties (101), or said distribution and
probability of expected local mechanical properties onto a Finite Element model (15) defining the geometry of said casting and said at least one evaluation area (13);
analysing said Finite Element model (15) using FEA analysis (16) for evaluating mechanical performance based on user requirements (11) defining local loads to be applied to said at least one evaluation area (13); and if necessary
adjusting said Finite Element model (15) or determining a set of adjusted input variables (17) to fulfil said user requirements (11).

9. The method according to any one of claims 1 to 4, the method further comprising:
obtaining a parametrised set of input variables (18) defining variations in a plurality of said input variables (1);
executing a Design of Experiments (19) by
executing multiple simulations (2) of the casting process using said numerical model (3) of said casting process for possible combinations of said parametrised set of input variables (18) to predict variations in local microstructures (6) and variations in local defects (21) at each part of the casting; and
determining a statistical strength assessment (22) for the parametrised set of input variables (18) in each part of the casting by
calculating variations in local microstructure based mechanical properties (8) at each part of the casting as a function of said variations in local microstructures (6);
calculating variations in local damage factors (9) at each part of the casting as a function of said variations in local defects (21); and
calculating variations in adjusted local mechanical properties (20) as a combined function of said variations in local microstructure based mechanical properties (8) and said variations in local damage factors (9).

10. The method according to claim 9, wherein the method further comprises determining a local defect probability (23) for the parametrised set of input variables (18) in each part of the casting based on said variations in local defects (21).

11. The method according to claim 9, wherein the method comprises a full factorial assessment by executing simulations (2) of the casting process for all possible combinations of the parametrised set of input variables (18).

12. The method according to claim 9, wherein the method comprises a limited assessment by applying a statistically supported reduction of the number of the parametrised set of input variables (18) and executing a number of simulations (2) of the casting process for a reduced parametrised set of input variables (18) and a set of fixed input variables (12).

13. The method according to any one of claims 9 to 12, further comprising determining information about process capability (24) for said parametrised set of input variables (18), for defining a statistical robustness of the casting process by determining at least one of:
a main effect and correlation diagram (100) describing the impact of varied input parameters on the cast part quality,
a minimum of expected mechanical properties (101) and a distribution of expected mechanical properties (102) of said casting based on predefined quality criteria, and
a robust process window (103) for said parametrised set of input variables (18).

14. The method according to claim 13, the method further comprising:
comparing at least one of said minimum of expected mechanical properties (101) and said distribution of expected mechanical properties (102) to predefined user requirements (11) regarding quality criteria of the casting; and
determining an adjusted parametrised set of input variables (39) to meet said user requirements (11).

15. The method according to any one of claims 1 to 14, the method further comprising:
obtaining at least one of said input variables (1), fixed input variables (12) or parametrised set of input variables (18) via an input-output interface (29) of said computing device (28); and
outputting the determined result of at least one step of the performed method to a display (30) of said computing device (28) .

16. A computer-based system comprising:
an input-output interface (29);
a display (30);
a non-transitory machine-readable storage medium (31) including a computer program product; and
at least one processor (32) operable to execute the program product, interact with the input-output interface (29) and the display (30), and perform operations according to the methods of any one of the claims 1 to 15.

17. A computer program product, encoded on a non-transitory machine-readable storage medium (31), operable to cause a processor (32) to perform operations according to the methods of any one of claims 1 to 15.
